Europäisches Patentamt

(19) European Patent Office

Office européen des brevets

(11) Publication number: **0 437 320 A1**

## (12) EUROPEAN PATENT APPLICATION

(21) Application number: **91300052.7**

(22) Date of filing: **04.01.91**

(51) Int. Cl.⁵: **C12N 15/17, C12N 5/10, C12N 15/82, C07H 21/04, C12P 21/02**

(30) Priority: **10.01.90 KR 26490**

(43) Date of publication of application:
**17.07.91 Bulletin 91/29**

(84) Designated Contracting States:
**DE FR GB**

(71) Applicant: **KOREA INSTITUTE OF SCIENCE AND TECHNOLOGY**
**39-1 Haweolgog-Dong, Sungbuk-Gu Seoul (KR)**

(72) Inventor: **Hong, Choo-Bong**
**186-1 Hongeun-Dong**
**Seodaemun-gu, Seoul (KR)**

Inventor: **Lee, Dae-Sil**
**Woo-sung APT 8-705, Gongneung-2-Dong**
**Nowon-Gu, Seoul (KR)**
Inventor: **Lee, Hye-Joo**
**Sorak APT 4-308, Jamwon-Dong**
**Seocho-Gu, Seoul (KR)**
Inventor: **Kim, Sung-Chun**
**19-4 Jongam-Dong**
**Sungbuk-Gu, Seoul (KR)**
Inventor: **Liu, Jang-Ryol**
**Woo-sung APT 6-802, 254, Gongneung-2-Dong**
**Nowon-Gu, Seoul (KR)**
Inventor: **Joung, Hyouk**
**Mi-sung APT 10-707, Wolgye-Dong**
**Nowon-Gu, Seoul (KR)**

(74) Representative: **Perry, Robert Edward et al**
**GILL JENNINGS & EVERY 53-64 Chancery Lane**
**London WC2A 1HN (GB)**

(54) **Recombinant DNA and its use in producing human insulin.**

㊗ Recombinant DNA comprising a plant chromosome and the human insulin gene provides plant cells and transgenic plants that can produce human insulin.

EP 0 437 320 A1

# RECOMBINANT DNA AND ITS USE IN PRODUCING HUMAN INSULIN

## Field of the Invention

This invention relates to recombinant DNA, and in particular to the engineering of transgenic tobacco plants for the production of insulin from plants.

## Background of the Invention

Insulin is produced from the pancreas in higher animals. It has a major function in carbohydrate metabolism. Any defect in insulin synthesis and function causes the most common adult disease, i.e. diabetes. Worldwide there are about one hundred and thirty million diabetics, and the number still increases.

Insulin as a pharmaceutical to treat diabetics has been mainly supplied after purification of insulin from pigs and cows. This has been insufficient to meet demand. Also, the product is not identical to human insulin, which results in low activity and some immunological response.

Recombinant DNA technology has made available the production of insulin in Escherichia coli, by manipulating the gene. The insulin gene which codes for the A, B and C chains of insulin, carried by an expression vector, has been introduced into E. coli. A prerequisite for this process is that the insulin gene must be fused to another gene coding for a protein. In other words, production of insulin in E. coli requires a large precursor protein which is a fused product of insulin and another protein. Otherwise, insulin is proteolytically degraded in E. coli (Steiner et al (1969) Recent Progress in Hormone Research 25 : 207-282 ; Wu et al (1984) Genetics: New Frontiers 2, Unipub. N.Y.).

Agrobacterium tumefaciens in conjunction with a binary vector can be used to transfer a foreign gene into plant chromosomes. The genes transferred and expressed in plants are those of prokaryotes and plants (Gasser et al (1989) Science 244 : 1293-1299). Recently, some animal protein genes have been successfully transferred into the chromosome of higher plants. Such products include the mouse metallothionein gene as a part of the tobacco chromosome (Maiti et al (1989) Plant Physiol. 91 : 1020-1024), the human enkephalin gene in the oilseed rape chromosome (Vandekerckhove et al (1989) Bio/Technology 7 : 929-932), and the immunoglobulin gene in the tobacco chromosome (Hiatt et al (1989) Nature 342 : 76-78).

## Summary of the Invention

The human insulin gene has been introduced into the tobacco chromosome, by using A. tumefaciens and a binary vector. The insulin protein produced in the transgenic tobacco has been found to be relatively stable.

## Description of the Invention

In the drawings :
Figure 1 shows a construction comprising a binary vector (pBKS1) into which the human insulin gene has been inserted ;
Figure 2 shows a restriction enzyme digest run on an agarose gel ;
Figure 3 shows the regeneration of a tobacco plant after transformation with the insulin gene (A : kanamycin-resistant calli formed on a leaf disc ; B : shoot formation from callus ; C : regenerated transgenic tobacco) ;
Figure 4 shows a Northern blot analysis for insulin transcripts in transgenic tobacco plants (Lanes 1 and 7 : RNA isolated from parental tobacco plants ; Lanes 2-6 : RNA isolated from transformed tobacco plants); and
Figure 5 shows a Western blot analysis for insulin produced in transformed tobacco plants (C : proteins from parental tobacco plants ; T : proteins from transformed tobacco plants).

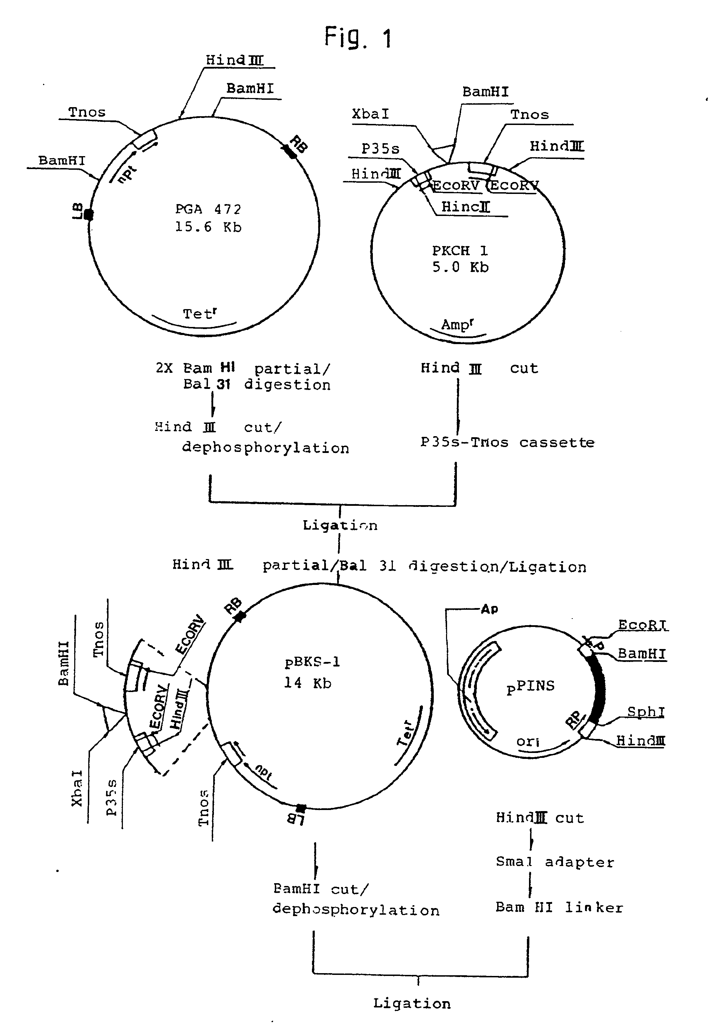

In order to produce a product in accordance with the invention, the human proinsulin gene was inserted into a higher plant transformation vector, pBKS-1. pBKS-1 is a recombinant DNA construct based on pGA 472 (provided by An, G., Washington State University, U.S.A. ; see An (1987) Methods in Enzymology 153 : 292-305) and pKCH-1 (Chung et al (1989) Korean J. Botany 32 : 23-32). The promoter DNA fragment of cauliflower mosaic virus 35S transcript (P35s) and the terminator DNA fragment of nopaline synthase gene (Tnos) of Ti (tumor-inducing) plasmid in pKCH-1 were isolated by HindIII restriction enzyme digestion. The P35s and Tnos fragment was then inserted into pGA 472 between LB (left border sequence of T-DNA of Ti plasmid) and RB (right border sequence of T-DNA of Ti plasmid). Thus the constructed pBKS-1 has all the components required for the transformation of higher plants. The coding sequence of human insulin protein, proinsulin type, was isolated from pPINS (provided by lee, D.S., Genetic Engineering Center, K.I.S.T., Korea) following Hind III restriction digest, Sma I adapter ligation, Bam HI linker ligation and Bam HI restriction digest. The isolated proinsulin coding sequence was inserted into the Bam HI site of pBKS-1 which is located between P35s and Tnos (see Figure 1).

The construct was introduced into A. tumefaciens, strain LBA 4404.

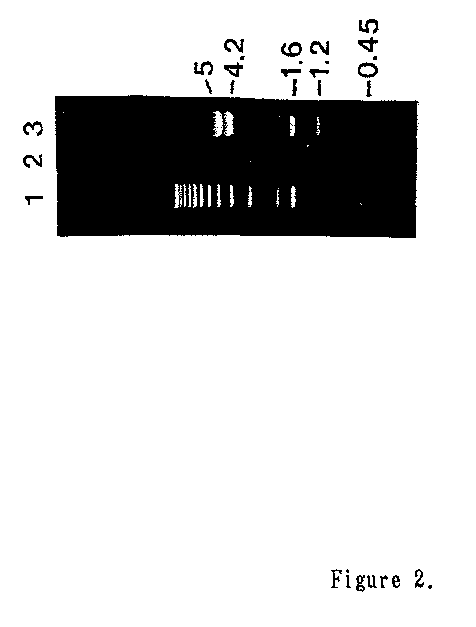

Introduction of the plasmid DNA into A. tumefaciens was carried out as An, G. (1987) with slight modifications. A. tumefaciens growing in YEP medium (10 g yeast extract, 10 g peptone, 5g NaCl per liter, pH 7.0) was harvested at log phase by centrifugation at 3,000 g for 5 min at 4 C. Bacterial pellet was resuspended in 20 mM ice cold CaCl2 solution. Plasmid DNA was added to the bacterial preparation, quickly frozen in liquid nitrogen and thawed at 37 C for 5 min. YEP medium was added and incubated at 28 C for 2-4 hours with shaking at 150 r.p.m. Bacteria carrying the insulin gene in pBKS-1 were selected on a tetracycline (5 ug/ml) containing LB-agar plate (Maniatis, T., Fritsch, E.F., and Sambrook, J. 1982. Molecular cloning. Cold Spring Harbor Laboratory. Cold Spring Harbor. N.Y.). Plasmid DNA was isolated by alkaline lysis method as described in Maniatis et al. (1982) and Hind III restriction digested. Restriction digests were run on an agarose gel in the presence of ethidium bromide. The bands seen at 5.0, 4.2, 1.6, 1.2 and 0.45 kbp indicate that the constructed DNA was introduced into A. tumefaciens correctly (see Figure 2).

Young and healthy leaves were collected from two months old tobacco plants (Nicotiana tabaccum cv. Wisconsin 38). Leaves were cut to squares and cocultured with A. tumefaciens carrying the insulin gene in pBKS-1 for 2 days in the dark at 25 C in callus induction medium*. After cocultivation, leaf pieces were washed with MS medium (Schuler, M.A. and Zielinski, R.E. 1989. Methods in Plant Molecular Biology. Academic Press, San Diego, U.S.A.) and cultured on a MS solid medium containing carbenicillin (250 ug/ml) and kanamycin (200 ug/ml). To induce shooting and rooting, shoot induction medium* and root induction medium* were used.

*callus induction medium : MS medium + 2 mg/l NAA + 0.5 mg/l BAP

*shoot induction medium : MS medium + 0.5 mg/l BAP

*root induction medium : MS medium + 1 mg/l NAA

(*BAP, 6-Benzylaminopurine : NAA, 1-Naphthalene acetic acid)

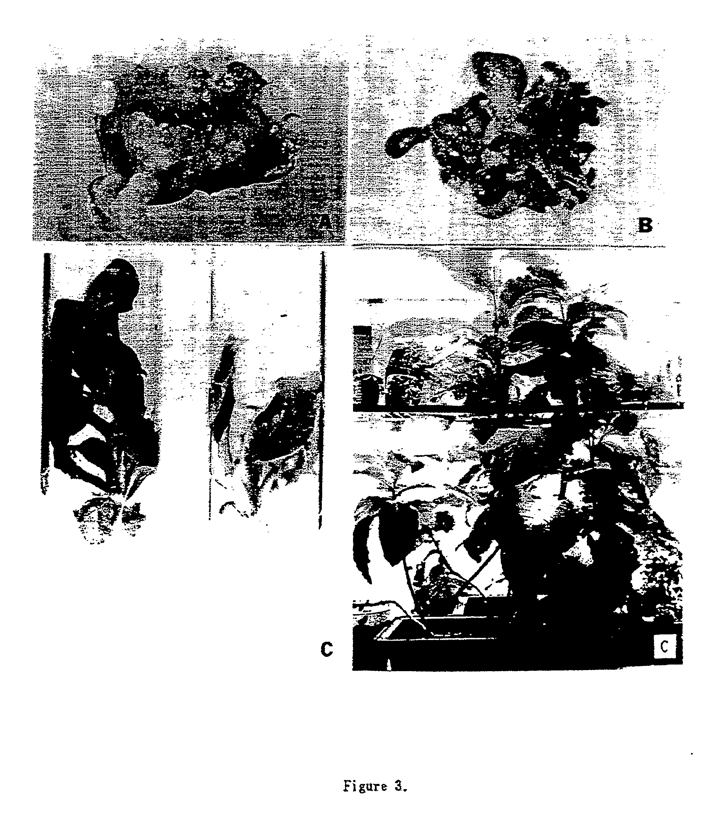

In two months, engineered transgenic tobacco plants with newly formed leaves and roots were well established (see Figure 3). The transformed tobacco plants were moved to soil and maintained in a green house.

Total RNA was prepared from the transformants on CsCl density gradient in the presence of guanidium thiocyanate (Hong, C.B. and Jeon, J.H. 1987. Korean J. Botany 30 : 201-203). RNA was size-fractionated on a 50 % formaldehyde - agarose gel and blotted onto Nytran membrane (Schleicher and Schuell, U.S.A.). Northern hybridization was carried out according to Maniatis et al. (1982). Labelling of the insulin DNA with 32P was done using T7 RNA polymerase after inserting the coding sequence of insulin in pGEM 3 (Promega, U.S.A.). Fifty percent of formamide at 37 C was the hybridization condition, and the membrane was washed with 0.1 x SSPE at 37 C for 2 hr after hybridization.

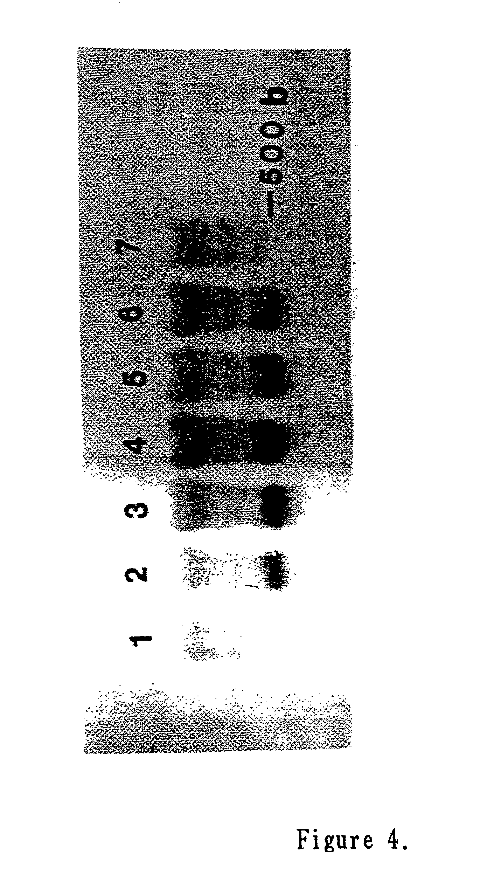

From the Northern hybridization, single major band was appeared at about 600 nucleotides from the transformed tobacco plants. Based on the size for the transcripts detected and absence of the band from the nontransgenic plants, it is conclusive that the insulin gene was successfully incorporated into tobacco chromosome and transcribed. The weak band at about 3.7 kb is due to the nonspecific hybridization of the probe to large ribosomal RNA. Hybridization was carried out at low stringency to induce nonspecitic hybridization on ribosomal RNA which was used as a standard to normalize the amount of RNA loaded on each well (see Figure 4).

Leaves of the putative transformed plants were quickly frozen in liquid nitrogen and ground in a mortar on ice. Proteins were dissolved in two volumes of sample buffer (0.125 M Tris pH 6.4, 4 % SDS, 20 % glycerol, 10 % beta-mercaptoethanol, 0.002 % bromophenol blue). The samples were then run on SDS-polyacrylamide gel electrophoresis. Proteins were blotted onto nitrocellulose membrane wetted with TBST butter (10 mM Tris pH 8.0, 150 mM NaCl, 0.05 % Tween 20). The membrane was prehybridized with 1 % bovine serum albumin and hybridized to anti-human insulin monoclonal antibody (Amersham, U.S.A., code RPN. 708) for 4-8 hr at room temperature. The membrane was washed with TBST buffer for 2 hr at room temperature. The membrane was again hybridized to anti-IgG alkaline phosphatase conjugate for 4 hr at room temperature and washed with TBST buffer for 2 hr at room temperature. Western bands for insulin protein from transgenic tobacco plants were visualized by staining the membrane with nitro blue tetrazolium and 5-bromo-4-chloro-3-indoyl phosphate as described by Amersham.

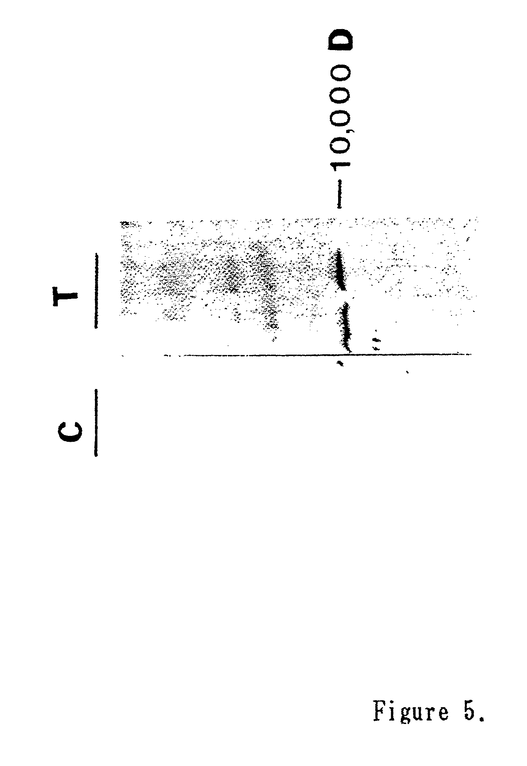

Specific Western bands were only seen from transgenic tobacco plants at about 10.000 D molecular weight. From the parental tobacco plants, no specific Western bands could be observed. Size of the protein detected on Western blotting and the specificity of Western blotting result confirm that transgenic plants carrying human insulin gene are producing insulin protein (see Figure 5). Based on the band intensity, it can be estimated that insulin was produced at the level of about 0.5 - 1.0 % of the total soluble protein of tobacco leaves.

Clearly the invention described here proves that transgenic tobacco plants are producing insulin. Thus transgenic tobacco plants can be used to manufacture insulin for the actual use. The procedure shown here can be used for other higher plants. Thus the invention described here can be applied to engineer insulin producing transgenic plants in general.

## EXAMPLE 1. CONSTRUCTION OF A HIGHER PLANT TRANSFORMATION VECTOR, pBKS-1, AND INSERTION OF HUMAN PROINSULIN GENE IN pBKS-1

Construction of pBKS-1 was based on two plasmids, pGA 472 and pKCH 1. pGA 472, provided by G.An at Washington State University, U.S.A., has the replication origin of E. coli and the wide host replication origin for the transformation process in Agrobacterium. It also has the border sequences of T-DNA, LB and RB, which are essential for the transfer of the target gene from Agrobacterium to the chromosome of higher plants. pGA 472 also has neomycin phosphotransferase gene for the selection of transformed plants and tetracycline resistance gene for the selection of transformed bacteria (An, G. 1987). pKCH 1 was constructed based on plasmid pUC 8. It has an ampicillin resistance gene, the promoter sequence of cauliflower mosaic virus 35S transcript (P35s) and the terminator sequence of nopaline synthase gene (Tnos) (Chung,S.H., Ko.K. W., Hong,C.B., Choi,I.S.,and Chung,T.W. 1989. Korean J. Botany 32 : 23-32). pGA 472 was partially digested with Bam HI to give one cut in it, and it produced a 15.6 kbp DNA fragment. The linearized pGA 472 was digested with Bal 31 exonuclease to remove one of the two Bam HI restriction sites in pGA 472 and ligated with T4 DNA ligase. Ligated DNA was introduced into E. coli, strain HB101, and thus amplified. Plasmid DNA amplified in E. coli was isolated by alkaline lysis method as described in Maniatis et al. (Maniatis,T., Fritsch,E. F., and Sambrook,J. 1982. Molecular cloning. Cold Spring Harbor Lab., Cold Spring Harbor, N.Y.). Plasmid DNA isolated was cut with Bam HI which resulted in an about 15 kbp linearized DNA and then digested with Bal 31 exonuclease. Second round Bal 31 exonuclease treatment removed the remaining Bam HI restriction site in pGA472. DNA was then ligated with T4 DNA ligase and introduced into E. coli to be amplified. Plasmid DNA was again isolated by alkaline lysis method and cut with Hind III restriction enzyme. The linearized DNA was about 13 kbp and its terminal phosphate groups were removed by alkaline dephosphatase. Plasmid pKCHI was cut with Hind III, and the 2 kbp DNA fragment was isolated by agarose gel electrophoresis and DEAE-cellulose paper elution (Maniatis et al., 1982). The 2 kbp DNA fragment contains P35s, Tnos and cloning sites, Bam HI and Xba I, for the insertion of a target gene (Chung,S.H. et al., 1989). The 2 kbp DNA fragment was ligated to the Hind III cut and dephosphorylated pGA 472 modified as described above. Recombinant DNA was then introduced into E. coli and amplified. This amplified DNA was cut with Hind III partially and Bal 31 digested to remove about 1 kbp flanking the Hind III site next of Tnos. A plasmid which satisfied above conditions was isolated and named pBKS-1. The size of pBKS-1 is about 14 kbp (Figure 1).

Proinsulin gene clone, pPINS, was provided by D.S.Lee (Genetic Engineering Center, K.I.S.T., Daejon, Korea). DNA sequence for proinsulin protein was synthesized using Beckman DNA synthesizer and carried by plasmid pUC 19 (Lee, D.S. et al., A study for human insulin production (III). Korean Ministry of Science and Technology Annual Report. pp. 24-28). For the cloning of proinsulin coding sequence in pBKS-1, pPINS was modified. pPINS was cut with Hind III, and Sma I adapter was linked using T4 DNA ligase. After amplification of the Sma I adapter linked pPINS in E. coli, the plasmid DNA was isolated and cut with Sma I. To the Sma I site, Bam HI linker was ligated using T4 DNA ligase and again amplified in E. coli. From this modified pPINS, the coding sequence for the B, C and A chains of proinsulin can be isolated using restriction enzyme Bam HI. Nucleotide sequence for 5′ terminal portion of proinsulin gene modified as described is as following,

```
5′GATCCGGGGGAGCTTGC----------proinsulin coding sequence----------------

  GCCCCCTCGAACG-------------------------------------------------------

 Bam HI

 linker



   Sma I

   adapter
```

From the modified pPINS, the coding sequence for proinsulin protein only was isolated by Bam HI restriction enzyme digestion, agarose gel electrophoresis and DEAE-cellulose papaer elution. Isolated proinsulin coding sequence was put to the Bam HI digested and dephosphorylated pPBKS-1 (Figure 1).

## EXAMPLE 2. TRANSFORMATION OF pBKS-1 CARRYING PROINSULIN GENE INTO AGROBACTERIUM TUMEFACIENS

Transformation of the plasmid constructed, pBKS-1 carrying proinsulin gene, into A. tumefaciens was carried out as described by An,G. (1987) with some modifications. A. tumefaciens, strain LBA 4404, was cullured to a log phase in YEP medium (10 g yeast extract, 10 g peptone, 5 g NaCl per liter, pH 7.0) al 28 C by shaking at 150 r.p.m. Bacterial cells were harvested by centrifugation at 3,000 g for 5 min at 4 C and resuspended in ice-cold 20 mM CaCl2 solution. Bacterial suspension was handled in about 7 ml volume glass culture tube, and the concentration of the bacterial suspension was about 1 x 10 cells/ml. About 0.5 ug of plasmid DNA was put to about 100 ul Agrobacterium suspension prepared as above, quickly frozen in liquid nitrogen and thawed in 37 C water bath for 5 min. About 1 ml of YEP medium was then added, and the cells were cultured for 2-4 hr at 28 C by shaking at 150 r.p.m.

Transformed Agrobacterium carrying pBKS-1 with proinsulin gene collected on an YEP-agar medium containing 5 ug/ml tetracycline. After two days incubation at 28 C, transformed bacterial colonies were identified and transferred to YEP liquid medium. After overnight incubation, bacterial cells were collected by centrifugation at 3,000 g for 5 min and plasmid DNA was isolated by alkaline lysis method (Maniatis et al., 1982). Plasmid DNA was digested with Pvu II and Hind III to confirm the pBKS-1 and proinsulin gene fusion in Agrobacterium. After restriction enzymes treatment. the digests were run on an agarose gel in the presence of ethidium bromide and viewed using ultra violet light illumination. From most of the bacterial cells collected on a tetracycline plate (more than 90 %), DNA fragments with the sizes of 5, 4.2, 1.6, 1.2, 0.45 kbp were identified which confirms the proper transformation of the plasmid in A. tumefaciens (Figure 2).

## EXAMPLE 3. TRANSFORMATION OF TOBACCO PLANTS (NICOTIANA TABACUM CV. WISCONSIN 38) WITH HUMAN PROINSULIN GENE AND IN VITRO REGENERATION OF TRANSFORMED TOBACCO PLANTS

Young leaves (between the eighth and third leaves from the top) from two months old tobacco plants grown in a green house were collected and immersed in 0.1 % hypochloride solution for 5 min to be sterilized. The leaves were cut to 1 x 1 cm , and the pieces were placed in callus-induction liqiud medium (*MS + 2 mg/l naphthaleneacetic acid + 0.5 mg/l 6-benzylamino purine) in a

*MS (Schuler and Zielinski, 1989. Methods in Plant Molecular Biology. Academic Press, San Diego, U.S.A.)

petri-dish. About 100 ul of A. tumefaciens with pBKS-1 and proinsulin gene grown to log-phase was added to the petri-dish which contains about 10 ml of callus-induction medium and ten pieces of tobacco leaves. They were incubated in the dark at 25 C for 1-2 days. The leaf pieces were washed with MS medium and placed on callus-induction agar medium (1.2 % agar was added) containing carbenicillin (250 ug/ml) and kanamycin (200 ug/ml). After incubation for two weeks on callus-induction agar medium, they were transferred onto shoot-induction medium (MS agar + 0.5 mg/l 6-benzyl aminopurine). In about three weeks on shoot-induction medium, usually four to five young leaves were developed. These young plantlets were then transferred onto root-induction medium (MS agar only), and in about three weeks roots were well developed. These small tobacco plants were moved to soil in a green house. In vitro regeneration processes were carried out at 25 C under 16 hr light and 8 hr dark period, and new medium was supplied every week (Figure 3).

## EXAMPLE 4. ASSAY FOR THE HUMAN PROINSULIN PRODUCING TRANSGENIC TOBACCO PLANTS

Northern blotting and Western blotting were performed to assay the putative human insulin producing transgenic tobacco plants.

Total RNA was isolaled from transgenic tobacco plants and nontransgenic tobacco plants, both are about two months old plants with more than ten leaves. About 2 g of leaves were harvested, washed with tap water, cut to pieces with scissors and ground in an ice-cold mortar after adding seven ml of lysis buffer. Lysis buffer contained 50 % guanidium thiocyanate, 0.5 % N-lauroyl sarcosine, 0.1 % beta-mercaptoethanol and 25 mM EDTA, pH 7.5, was cooled on ice before use. After grinding for five minutes, the ground was filtered through one layer of mira cloth. The filtrate was centrifuged at 6,000 x g for 10 min at 4 C, and the supernatant was placed on the cushion of 5.7 M CsCl in 0.1 M EDTA (pH 7.5) solution. The filtrate and the cushion were used at the equal amounts. It was then centrifuged for 22 hr at 15 C in SW 50.1 rotor at 22,000 r.p.m. Precipitate was dissolved in 10 mM Tris.Cl,pH 7.5, containing 0.1 % SDS and extracted with chloroform and 1-butanol (1:4) mixture. RNA was collected from the aqueous phase by ethanol precipitation (Hong,C.B. and Jeon,J.H. 1987. Korean J. Botany. 30 : 201-203). RNA was size-fractionated by running 50 % formaldehyde agarose gel electrophoresis. After electrophoresis, the gel was washed with distilled water for three times and soaked in 20 x SSPE. RNA was transferred onto Nytran membrane (Maniatis et al., 1982). After transfer, Nytran membrane was irradiated with U.V. for 5 min and air-dried for two hours. Northern hybridization condition was 50 % formamide and 37 C overnight, and washing was carried out with 0.1 x SSPE at 37 C for two hours. Proinsulin gene probe was prepared after putting the proinsulin coding sequence isolated from the modified pPINS with Bam HI into the plasmid vector pGEM 3 (Promega, U.S.A.). Using SP 6 RNA polymerase the sequence was 32P labelled using 32P-UTP. Other conditions were as described in Maniatis et al. (1982). After washing the membrane was wrapped with vinyl wrap and exposed to X-ray film at -70 C with two intensifying screens.

From the transgenic plants, unique Northern band of about 500-600 bases was identified while non-transgenic plants dit not have any (Figure 4). These Northern results clearly indicate that the transformation processes were successful to introduce the human insulin gene into tobacco chromosome and to lead to the production of insulin transcripts.

Western blotting was carried out to assay the production of proinsulin protein in the transgenic tobacco plants. From the transgenic plants at which synthesis of insulin transcripts was confirmed , two grams of leaves were collected and ground in an ice-cold mortar as described for the purification of RNA. For the nontransgenic plants, leaves were ground likewise. Lysis buffer used for extraction of protein was 0.125 M Tris•Cl, pH 6.8, 4 % SDS, 20 % glycerol, 10 % beta-mercaptoethanol, 0.002 % bromophenolblue, and about three volumes of lysis buffer was used for the leaf samples. After addition of the extraction buffer, the sample was boiled at 100 C for 5 min, centrifuged at 10,000 r.p.m. for 20 min in an Eppendorf microcentrifuge, and the supernatant was collected. The supernatant was loaded on a 15 % SDS-polyacrylamide gel and electrophoresis was carried out at 15-20 mA for 2-3 hr at room temperature. After electrophoresis, proteins were blotted onto nitrocellulose membrane wet with TBST buffer (10 mM Tris•Cl, pH 8.0, 150mM NaCl, 0.05 % Tween 20) by electrophoresis at 1.5 amp for 1-2 hr in 1 x TAE buffer (Sambrook,J., Fritsch,E.F., and Maniatis,T. 1989. Molecular Cloning. 2nd ed. Cold Spring Harbor Lab., Cold Spring Harbor, U.S.A.). Nitrocellulose membrane was prehybridized with 1 % bovine serum albumin solution to block nonspecific hybridization and hybridized with anti-human insulin monoclonal antibody (Amersham, U.S.A., #RPN. 708). Western hybridization with the monoclonal antibody was carried out for 4-8 hr at room temperature, and the membrane was washed with TBST at room temperature for 1 hr. The membrane was then hybridized with anti-IgG and alkaline phosphatase conjugate for 1 hr at room temperature and again washed with TBST at room temeprature for 30 min. Nitroblue tetrazolium and 5-bromo-4-chloro-3-indoyl phosphate were added for colouring as described by the manufacturer (Amersham, U.S.A.). A unique Western band, corresponding to about 10,000 D, was observed for the transformed plants but not for the non-transgenic plants (Figure 5).

The transgenic Nicotiana tabacum tissue in accordance with the invention (for which results are shown in Fig. 5) has been desposited at the Korean Collection for Type Cultures. The deposit date and accession number are 01.12.90 and KCTC 0002BP.

"K.I.S.T" = Korea Institute of Science & Technology.

## Claims

1. Recombinant DNA comprising a plant chromosome and the human insulin gene.

2. Recombinant DNA according to claim 1, wherein the plant is the tobacco plant.

3. A process for producing recombinant DNA according to claim 1 or claim 2, which comprises transferring the human insulin gene into the plant chromosome by means of Agrobacterium tumefaciens and a binary vector.

4. Plant cells characterised by the ability to produce human insulin.

5. Plant cells according to claim 4, wherein the plant is the tobacco plant.

6. Plant cells according to claim 4 or claim 5, in the form of callus or other tissue.

7. A transgenic plant characterised by the ability to produce human insulin.

8. A transgenic plant according to claim 7, wherein the plant is the tobacco plant.

9. A method for producing human insulin, which comprises culturing plant cells according to any of claims 4 to 6.

# Fig. 1

Figure 2.

Figure 3.

Figure 4.

Figure 5.

European Patent
Office

**EUROPEAN SEARCH REPORT**

Application Number

| | DOCUMENTS CONSIDERED TO BE RELEVANT | | EP 91300052.7 | |
|---|---|---|---|---|
| Category | Citation of document with indication, where appropriate, of relevant passages | Relevant to claim | CLASSIFICATION OF THE APPLICATION (Int. Cl.5) | |
| X | WO - A1 - 84/02 920 (MONSANTO COMPANY) * Claims 1,5,13,15,17; page 3, lines 1-5; page 12, lines 13-24 * | 1-9 | C 12 N 15/17 C 12 N 5/10 C 12 N 15/82 C 07 H 21/04 C 12 P 21/02 | |
| D,A | PLANT PHYSIOLOGY, vol. 91, no. 3, November 1989, New York I.B. MAITI et al. "Inheritance and Expression of the Mouse Metallothionein Gene in Tobacco" pages 1020-1024 * Totality * | 1-9 | | |
| D,A | BIOTECHNOLOGY, vol. 7, no. 9, September 1989, New York J. VANDEKERCKHOUE et al. "Enkephalins Produced in Transgenic Plants Using Modified 2S Seed Storage Proteins" pages 929-932 * Totality * | 1-9 | TECHNICAL FIELDS SEARCHED (Int. Cl.5) C 12 N C 07 H C 12 P | |
| D,A | NATURE, vol. 342, no. 6245, November 2, 1989, London A. HIATT et al. "Production of antibodies in transgenic plants" pages 76-78 * Totality * | 1-9 | | |

The present search report has been drawn up for all claims

| Place of search | Date of completion of the search | Examiner |
|---|---|---|
| VIENNA | 12-03-1991 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)